# EUROPEAN PATENT SPECIFICATION

(11) **EP 4 009 877 B1**
(45) Date of publication and mention of the grant of the patent: **07.06.2023**
(21) Application number: 20757109.2
(22) Date of filing: 29.07.2020
(51) Int. Cl.: A61B 10/00, A61F 5/44, A61F 5/453, A61F 5/455

(54) **FLUID COLLECTION ASSEMBLIES INCLUIDNG A SAMPLE PORT**
FLUIDSAMMELANORDNUNGEN MIT EINEM PROBENPORT
ENSEMBLES DE COLLECTE DE FLUIDE COMPRENANT UN ORIFICE D'ÉCHANTILLON

(30) Priority: 06.08.2019 US 201962883172 P
(43) Date of publication of application: 15.06.2022
(60) Divisional application: 23166972.2
(73) Proprietor: Purewick Corporation, El Cajon, California 92020 (US)
(72) Inventor: JOHANNES, Ashley Marie, Atlanta, Georgia 30340 (US)
(74) Representative: Hoffmann Eitle
(86) International application number: PCT/US2020/044024
(87) International publication number: WO 2021/025919

(56) References cited:
- US-A- 4 747 166
- US-A1- 2017 266 031
- US-A1- 2017 325 788

## Description

### BACKGROUND

A person or animal may have limited or impaired mobility such that typical urination processes are challenging or impossible. For example, a person may experience or have a disability that impairs mobility. A person may have restricted travel conditions such as those experienced by pilots, drivers, and workers in hazardous areas. Additionally, sometimes urine collection is needed for monitoring purposes or clinical testing.

Urinary catheters, such as a Foley catheter, can be used to address some of these circumstances, such as incontinence. Unfortunately, urinary catheters can be uncomfortable, painful, and can lead to complications, such as infections. Additionally, bed pans, which are receptacles used for the toileting of bedridden patients are sometimes used. However, bedpans can be prone to discomfort, spills, and other hygiene issues.

### SUMMARY

The invention is defined in claim 1 below, in which the pre-characterizing part is formulated to conform to the disclosure of US 2017/0266031 A1. The dependent claims are directed to optional features and preferred embodiments. In an embodiment, a fluid collection assembly is disclosed. The fluid collection assembly includes a fluid impermeable barrier. The fluid impermeable barrier includes at least one interior surface defining a chamber. The fluid impermeable barrier defines at least one opening and a barrier outlet. The fluid collection assembly includes at least one wicking material disposed in the chamber. The fluid collection assembly also includes a sample port defining at least one channel. The at least one channel defining and extending between at least a port inlet and an attachment port. The attachment port is configured to switch between a closed state when a flow of bodily fluids through the attachment port is restricted and an open state when the flow of the bodily fluids through the attachment port is permitted. The fluid collection assembly further includes a sample container defining a sample compartment and a sample inlet configured to couple with the attachment port. The sample container includes a check valve between the sample inlet and the sample compartment.

In an embodiment, a fluid collection system is disclosed. The fluid collection system includes a fluid collection assembly. The fluid collection assembly includes a fluid impermeable barrier. The fluid impermeable barrier includes at least one interior surface defining a chamber. The fluid impermeable barrier defines at least one opening and a barrier outlet. The fluid collection assembly includes at least one wicking material disposed in the chamber. The fluid collection assembly also includes a sample port defining at least one channel. The at least one channel defining and extending between at least a portion inlet and an attachment port. The attachment port is configured to switch between a closed state when a flow of bodily fluids through the attachment port is restricted and an open state when the flow of the bodily fluids through the attachment port is permitted. The fluid collection assembly further includes a sample container defining a sample compartment and a sample inlet configured to couple with the attachment port. The sample container includes a check valve between the sample inlet and the sample compartment. The fluid collection system also includes a fluid storage container positioned downstream from the fluid collection assembly and a vacuum source positioned downstream from the fluid storage container.

In an embodiment, a method, not part of the claimed invention, of collecting a sample of bodily fluids is disclosed. The method includes positioning at least one opening defined by a fluid impermeable barrier of a fluid collection assembly such that the at least one opening is positioned adjacent to or receives a urethra of an individual. The fluid impermeable barrier includes at least one interior surface defining a chamber. The method also includes receiving the bodily fluids from the urethra through the at least one opening and into at least one wicking material disposed in the chamber. The method additionally includes removing at least some of the bodily fluids from the chamber via a barrier outlet defined by the fluid impermeable barrier. Further, the method includes receiving at least a portion of the bodily fluids into a port inlet defined by a sample port, through at least one channel, and into a sample compartment defined by a sample container. The at least one channel defines and extends between at least the port inlet and the attachment port. The port inlet is in fluid communication with the chamber, the attachment port is configured to switch between a closed state when the flow of the bodily fluids through the attachment port is restricted and an open state when the flow of the bodily fluids through the attachment port is permitted, and the sample container defines a sample inlet configured to couple with the attachment port. The sample container includes a check valve between the sample inlet and the sample compartment.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1A** is a schematic isometric view of a fluid collection assembly, according to an embodiment.
**FIG. 1B** is a cross-sectional view of the fluid collection assembly, according to an embodiment.
**FIG. 2A** is a schematic isometric view of a fluid collection assembly that includes a sample port integrally formed with a fluid impermeable barrier, according to an embodiment.
**FIG. 2B** is a schematic cross-sectional view of the fluid collection assembly, according to an embodiment.
**FIG. 3A** is a schematic isometric view of a fluid collection assembly that includes a sample port integrally formed with a fluid impermeable barrier, according to an embodiment.
**FIG. 3B** is a schematic cross-sectional view of the fluid collection assembly, according to an embodiment.
**FIG. 4** is a schematic cross-sectional view of a fluid collection assembly defining an opening that is configured to receive a urethra, according to an embodiment.
**FIG. 5** is a schematic illustration of fluid collection system that includes a fluid collection assembly, according to an embodiment.
**FIG. 6** is a flow chart of a method for collecting a sample of bodily fluids, according to at least some embodiments.

### DETAILED DESCRIPTION

Fluid collection assemblies including a sample port, systems including the same, and methods of using the same are disclosed herein. An example fluid collection assembly includes a fluid impermeable barrier defining at least one opening and a chamber. The fluid collection assembly also includes at least one wicking material disposed in the chamber. The fluid collection assembly additionally includes a sample port and a sample container that is configured to be coupled to the sample port. The sample port and the sample container allow a sample of the bodily fluids (*e.g.,* urine) to be obtained.

During use, the opening of the fluid impermeable barrier is positioned adjacent to or receives the urethra of an individual. Bodily fluids that are emitted from the urethra are received by the wicking material of the fluid collection assembly. The bodily fluids are removed from the chamber using at least one tube that is in fluid communication with the chamber by applying a suction force from the tube to the chamber.

With conventional catheters (*e.g.,* Foley catheters), samples may be obtained by disposing a syringe or syringe like device into a port that is in fluid communication with a tube. The syringe or syringe like device may then remove a sample of the bodily fluids by suctioning the bodily fluids that are present in the tube or that are present in the tube after kinking the tube. However, such methods of obtaining samples from the fluid collection assemblies disclosed herein can be difficult and/or impossible for at least one of several reasons. First, conventional catheters may slowly remove bodily fluids from the individual in a continuous or semi-continuous manner such that bodily fluids are generally present in the tube and/or may quickly pool after kinking the tube. However, the fluid collection assemblies disclosed herein may only receive the bodily fluids when the individual urinates. This may require a medical practitioner (*e.g*., nurse) to wait prolonged periods of time between urination, especially when the individual is unable to control urination. Second, the suction force may move the bodily fluids quickly through the tube. The quick movement of the bodily fluids may make removing the bodily fluids from the tube using a syringe or syringe-like device difficult and may only provide the medical practitioner a short period of time to remove the bodily fluids. Third, unlike conventional catheters, the tube should not be kinked since kinking the tube would prevent the suction force from removing the bodily fluids from the chamber. Failing to promptly remove the bodily fluids from the chamber with the suction force may cause the chamber to become filled with the bodily fluids and cause the bodily fluids to leak from the chamber.

The sample ports and the sample containers disclosed herein remedy at least some of the problems associated with obtaining samples of the bodily fluids from the fluid collection assemblies disclosed herein using syringes or syringe-like device. For example, the sample port includes an attachment port and the sample container includes a sample inlet that may be coupled to the attachment port attachment port when a sample of the bodily fluids is desired. The sample port is positioned such that at least some of bodily fluids that are discharged from the urethra of the individual come in contact with the attachment port. At least some of the bodily fluids that come in contact with the attachment port may flow from the attachment port and into the sample container. The sample container includes a check valve that allows the bodily fluids to flow into a sample compartment defined by the sample container. The check valve also inhibits removal of the bodily fluids from the sample compartment. Once a sufficient quantity of the bodily fluids are present in the sample compartment, the sample container may be decoupled from the sample port and, as such, a sample has been obtained from the fluid collection assembly. The attachment port may be configured to switch to a closed state when the sample container is decoupled therefrom thereby preventing or at least inhibiting any bodily fluids leaking from the sample port.

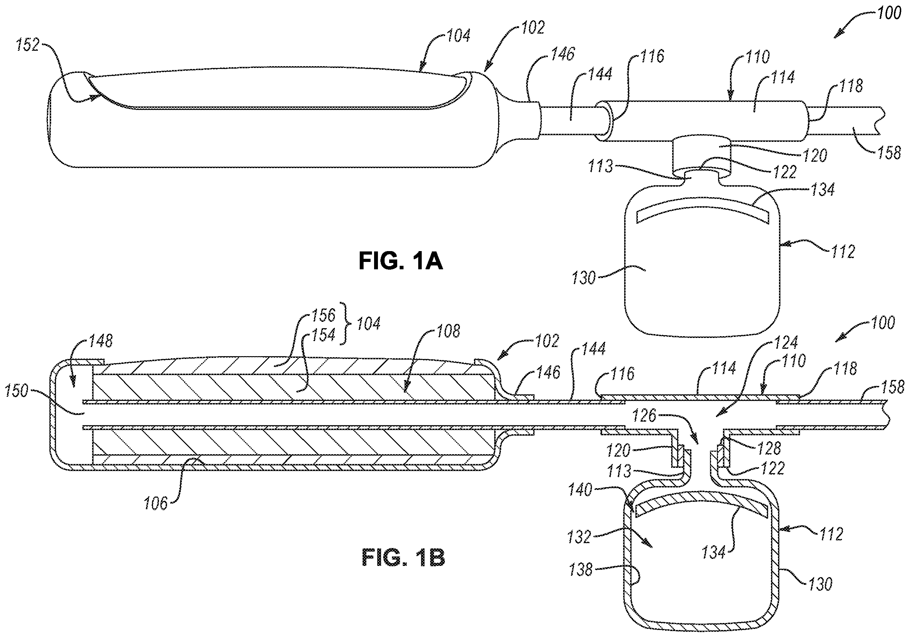

**FIG. 1A** is a schematic isometric view of a fluid collection assembly 100, according to an embodiment. **FIG. 1B** is a cross-sectional view of the fluid collection assembly 100, according to an embodiment. The fluid collection assembly 100 includes a fluid impermeable barrier 102 and at least one wicking material 104 disposed in the fluid impermeable barrier 102. For example, the fluid impermeable barrier 102 includes at least one interior surface 106 defining a chamber 108 and the at least one wicking material 104 is disposed in the chamber 108. The fluid collection assembly 100 also includes a sample port 110 that is distinct from and, optionally, spaced from the fluid impermeable barrier 102. The fluid collection assembly 100 may also include a sample container 112. The sample container 112 is couplable to the sample port 110 and, as illustrate, may actually be coupled to the sample port 110. For example, the sample container 112 includes a sample inlet 113 that is configured to be coupled to an attachment port 122 of the sample port 110.

The sample port 110 includes at least one first wall 114 that defines a port inlet 116 and a port outlet 118 opposite the port inlet 116. In the illustrated embodiment, the first wall 114 exhibits a generally cylindrical shape which may facilitate attachment of the port inlet 116 and the port outlet 118 to cylindrical tubes (*e.g.,* first tube 144 and/or second tube 158) though the first wall 114 may exhibit other suitable shapes. In an embodiment, as illustrated, the sample port 110 may also include at least one second wall 120 extending from the first wall 114. The second wall 120 may define the attachment port 122. The second wall 120 exhibits a generally cylindrical shape which may facilitate attachment of the attachment port 122 to a cylindrical sample inlet 113 though the second wall 120 may exhibit other suitable shapes. In an embodiment, the sample port 110 does not include the second wall 120. In such an embodiment, the first wall 114 may form the attachment port 122.

Referring to **FIG. 1B****,** the first wall 114 defines a first channel 124 extending from the port inlet 116 to the port outlet 118. The first channel 124 allows bodily fluids to flow from the port inlet 116 to the port outlet 118. The second wall 120 defines a second channel 126 extending from the first channel 124 to the attachment port 122. The second channel 126 allows at least some of the bodily fluids flowing through the first channel 124 to be diverted and reach the attachment port 122. As previously discussed, at least some of the bodily fluids that reach the attachment port 122 may enter the sample container 112.

The attachment port 122 is configured controllable switch between a closed state and an open state. The attachment port 122 restricts the flow of bodily fluids through the attachment port 122 when the attachment port 122 is in the closed state and permits the bodily fluids to flow through the attachment port 122 when the attachment port 122 is in the open state. Generally, the attachment port 122 is in the closed state when the sample container 112 is not coupled to the attachment port 122 to prevent bodily fluids from leaking from the sample port 110. However, the attachment port 122 is generally in the open state when the sample container 112 is coupled to the attachment port 122. In an embodiment, the attachment port 122 may be configured to switch from the closed state to the open state by coupling the sample container 112 to the attachment port 122 and switched from the open state to the closed state by decoupling the sample container 112 from the attachment port 122. However, it is noted that the attachment port 122 may switch between the closed and open states using techniques other than coupling and decoupling the sample container 112 to the attachment port 122. For example, the attachment port 122 may include or be operably coupled to an actuator which, responsive to direction from a user *(e.g.,* the individual using the fluid collection assembly 100 or the medical practitioner) opens and closes a mechanical valve disposed in the attachment port 122.

The attachment port 122 includes a valve 128, which is shown schematically in **FIG. 1B****.** The valve 128 is configured to switch the attachment port 122 between the closed state (*e.g.,* the valve 128 is closed) to the open state (*e.g.,* the valve 128 is open). In an embodiment, the valve 128 is a mechanical valve, such as ball valve, a butterfly valve, a choke valve, etc. In an embodiment, the valve 128 includes a sealing device bonded to or otherwise attached to the second wall 120 (*e.g.,* the second wall 120 is a rigid wall). The sealing device may define a through hole that is initially closed in an at least substantially fluid tight manner when the sample container 112 is not coupled to the attachment port 122. The sealing device may stretch and/or conform to the sample inlet 113 of the sample container 112 when the sample container 112 is coupled to the attachment port 122 thereby enlarging the through hole. As such, the sealing device may form an at least substantially fluid tight seal when the sample container 112 is coupled to the attachment port 122. The sealing device may be configured to reseal itself in an at least substantially fluid tight manner after the sample container 112 is detached from the attachment port 122. In an embodiment, the valve 128 and the sample inlet 113 of the sample container 112 form a luer lock or luer slip connection. In such an embodiment, one of the valve 128 or the sample inlet 113 may form the male fitting of the luer lock or luer slip connection while the other of the valve 128 or the sample inlet 113 forms the female fitting of a luer lock or luer slip connection. In an embodiment, the valve 128 may be configured to receive a syringe that includes a needle.

In an embodiment, the sample port 110 includes at least one seal extending across at least one of the port inlet 116, the port outlet 118, or the attachment port 122. The seal may prevent microbes from entering the sample port 110 (*e.g.,* entering the first channel 124 or the second channel 126). The seal may also indicate whether the sample port 110 has been tampered with or previously used. For example, the sample port 110 may be provided with the seal intact. However, connecting the port inlet 116 to the first tube 144, connecting the port outlet 118 to the second tube 158, connecting the attachment port 122 to the sample inlet 113, or otherwise accessing the interior of the sample port 110 may break or otherwise damage the seal thereby indicating that the sample port 110 was tampered with or previously used. In an example, the act of at least one of connecting the port inlet 116 to the first tube 144, connecting the port outlet 118 to the second tube 158, or connecting the attachment port 122 to the sample inlet 113 may break or otherwise damage the seal.

The sample container 112 may include any suitable container that can receive and hold bodily fluids and is couplable to the attachment port 122. In the illustrated embodiment, the sample container 112 includes one or more panels 130. The one or more panels 130 define the sample inlet 113 and the sample compartment 132. The one or more panels 130 may be formed from a flexible or rigid material. The one or more panels 130 may also be transparent which allows the presence of bodily fluids in the sample compartment 132 to be easily detected. However, the one or more panels 130 may be opaque without limitation. For example, a portion of the one or more panels 130 may be transparent while an opposing portion of the one or more panels 130 is opaque which may improve the contrast between the bodily fluids and the background. In an embodiment, the one or more panels 130 may include one or more volumetric markings thereon. The volumetric markings may be used to determine the quantity of the bodily fluids that are present in the sample compartment 132.

The sample container 112 may include a check valve 134. The check valve 134 is positioned between the sample inlet 113 and the sample compartment 132. The check valve 134 is configured to permit bodily fluids to flow from the sample inlet 113 into the sample compartment 132 and to restrict the flow of the bodily fluids from the sample compartment 132 to the sample inlet 113. As such, the check valve 134 prevents or at least inhibits a suction force from removing the bodily fluids from the sample compartment 132.

The check valve 134 may include any suitable check valve. In the illustrated embodiment, the check valve 134 includes a curved barrier. The curved barrier substantially extends from a portion of at least one interior surface 138 to an opposing portion of the of the interior surface 138 such that only a small gap 140 is present between the curved barrier and the interior surface 138. Further, the curved barrier is convexly curved relative to the sample inlet 113 may also encourage the bodily fluids to flow towards the gap 140. However, the small dimension of the gap 140, the concave curvature of the curved barrier relative to the sample compartment 132, and the tortuous path formed by the curved barrier inhibits flow of the bodily fluids from the sample compartment 132 to the sample inlet 113.

As previously discussed, the sample port 110 is spaced from the fluid impermeable barrier 102. As such, the fluid collection assembly 100 may include at least one first tube 144 that is coupled to and extends between the sample port 110 and the fluid impermeable barrier 102. In particular, the first tube 144 may extend between and be coupled to the port inlet 116 and an barrier outlet 146 defined by the fluid impermeable barrier 102. The first tube 144 may be coupled to the port inlet 116 and the barrier outlet 146 using any suitable method. In an example, a portion of the first tube 144 may be disposed in at least one of the port inlet 116 or the barrier outlet 146 and maintained therein via an interference fit. In an example, at least one of the port inlet 116 or the barrier outlet 146 may be disposed in the first tube 144 and maintained therein via an interference fit. In an example, the first tube 144 may be coupled to the port inlet 116 or the barrier outlet 146 via a threaded connection.

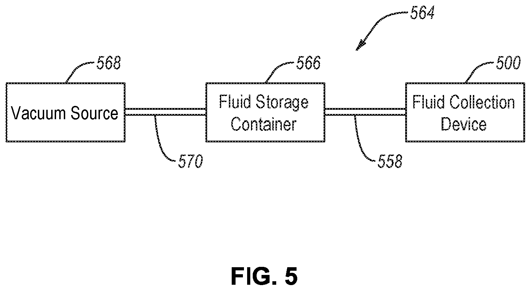

In an embodiment, as illustrated, the at least one tube 144 is configured (*e.g.,* exhibits a length) such that the sample port 110 is positioned relatively close to the barrier outlet 146. For example, the tube 144 may be configured such that that sample port 110 is positioned within about 100 cm of the barrier outlet 146, such as within about 75 cm, within about 50 cm, within about 25 cm, within about 10 cm, within about 5 cm, or ranges of about 2.5 cm to about 10 cm, about 5 cm to about 25 cm, about 10 cm to about 50 cm, about 25 cm to about 75 cm, or about 50 cm to about 100 cm of the barrier outlet 146. However, the at least one tube 144 may be configured (*e.g*., exhibits a length) such that the sample port 110 is spaced from the barrier outlet 146 may a distance greater than 100 cm. For example, the at least one tube 114 may be configured such that the sample port 110 is closer to a fluid storage container (*e.g.,* fluid storage container 566 of **FIG. 5**) than the barrier outlet 146. For instance, the at least one tube 114 may be configured such that the sample port 110 is within 100 cm of the fluid storage container.

In the illustrated embodiment, referring to **FIG. 1B****,** the first tube 144 extends through the barrier outlet 146 of the fluid impermeable barrier 102 such that at least a portion of the first tube 144 is disposed in the chamber 108 defined by the fluid impermeable barrier 102. Disposing the first tube 144 to be in the chamber 108 may facilitate removal of any bodily fluids in the chamber 108. For example, as will be discussed in more detail below, the bodily fluids that enter the chamber 108 may be wicked towards a fluid reservoir 148. Disposing the first tube 144 such that a tube inlet 150 of the first tube 144 is positioned in or near the fluid reservoir 148 may facilitate removal of the bodily fluids from the chamber 108.

In an embodiment (not shown), the first tube 144 and the sample port 110 are integrally formed together such that the first tube 144 forms part of the sample port 110. In such an embodiment, the sample port 110 is not spaced from the fluid impermeable barrier 102 but is instead coupled to the barrier outlet 146 of the fluid impermeable barrier 102. Further, a portion of the sample port 110 may be disposed in the chamber 108. For example, the sample port 110 may be disposed in the chamber 108 such that the port inlet 116 is positioned in or near the fluid reservoir 148.

The fluid impermeable barrier 102 defines at least one opening 152 (*e.g.,* an elongated opening). The opening 152 is configured to be positioned adjacent to a urethra, such as a female urethra or a buried male urethra. As such, the opening 152 allows bodily fluids discharged from the urethra to enter the chamber 108. The fluid impermeable barrier 102 also defines a substantially unoccupied fluid reservoir 148 (shown in **FIG. 1B**). The fluid impermeable barrier 102 may be formed from silicone, another thermosetting polymer, another suitable fluid impermeable material, or combinations thereof.

As previously discussed, the fluid collection assembly 100 includes at least one wicking material 104. In the illustrated embodiment, referring to **FIG. 1B****,** the wicking material 104 includes a fluid permeable support 154 and a fluid permeable membrane 156. The fluid reservoir 148, the fluid permeable support 154, and the fluid permeable membrane 156 may be arranged such that any bodily fluids flowing through the opening 152 flows through the fluid permeable membrane 156, through the fluid permeable support 154, and into the fluid reservoir 148. The bodily fluids may then flow from the fluid reservoir 148 to the first tube 144 thereby removing the bodily fluids from the chamber 108.

The fluid permeable support 154 can be positioned relative to the permeable membrane 156 such that the fluid permeable support 154 maintains the permeable membrane 156 in a particular shape. In an embodiment, the fluid permeable support 154 can be configured to maintain the permeable membrane 156 against or near the urethral of the individual. For example, the fluid permeable support 154 can include a portion having a curved shape in contact with the permeable membrane 156 such that the permeable membrane 156 is also curved, thus creating a comfortable and secure interface for engagement with the urethral and/or the area of the body near the urethral.

In an embodiment, the fluid permeable support 154 can be made of a rigid plastic. In an embodiment, the fluid permeable support 154 can have any suitable shape and be formed of any suitable material. For example, the fluid permeable support 154 can be flexible. Additionally, the fluid permeable support 154 can be formed of aluminum, a composite of plastic and aluminum, some other metal and/or a composite. In an embodiment, the fluid permeable support 154 can be formed of a natural material, such as, for example, plant fibers (e.g., Greener Clean manufactured by 3M^{®}). The natural material can include openings that allow fluid to flow through the natural material. In an embodiment, the fluid permeable support 154 can be cylindrical and can define a lumen. In an embodiment, the fluid permeable support 154 can be formed of perforated coated paper, such as tubular waxed paper. In an embodiment, the fluid permeable support 154 may be formed from spun plastic, such as non-woven permeable nylon and/or polyester webbing.

The fluid permeable membrane 156 can be formed of a material that is permeable to the bodily fluids and has wicking properties. The fluid permeable membrane 156 can have a high absorptive rate and a high permeation rate such that the bodily fluids can be rapidly absorbed by the fluid permeable membrane 156 and/or transported through the fluid permeable membrane 156. In an embodiment, the fluid permeable membrane 156 can be a ribbed knit fabric. In an embodiment, the fluid permeable membrane 156 can include and/or have the moisture-wicking characteristic of gauze, felt, terrycloth, thick tissue paper, and/or a paper towel. In an embodiment, the fluid permeable membrane 156 can be soft and/or minimally abrasive such that the fluid permeable membrane 156 does not irritate the skin of the user. The fluid permeable membrane 156 can be configured to wick bodily fluids away from the urethral and/or the skin of the individual such that the dampness of the skin of the individual is lessened and infections are prevented. Additionally, the wicking properties of the fluid permeable membrane 156 can help prevent bodily fluids from leaking or flowing beyond the assembly onto, for example, a bed. In an embodiment, the fluid permeable membrane 156 can be formed of fine denier polyester fibers coated with a thermoplastic water-based binder system.

In an embodiment, the at least one of the fluid permeable support 154 or the fluid permeable membrane 156 may be omitted from the fluid collection assembly 100 such that the wicking material 104 only includes a single material. In an embodiment, the wicking material 104 may include three or more materials, such as the fluid permeable support 154, the fluid permeable membrane 156, and at least one additional material. Regardless, one or more components of the wicking material 104 may include permeable material designed to wick or pass fluid therethrough. The permeable properties referred to herein may be wicking, capillary action, diffusion, or other similar properties or processes, and are referred to herein as "permeable" and/or "wicking." Such "wicking" may not include absorption of one or more components of the bodily fluids *(e.g*., urine) into the one or more components of the wicking material 104. Put another way, substantially no absorption of one or more components of the bodily fluids into the one or more components of the wicking material 104 may take place after the wicking material 104 is exposed to the bodily fluids. While no absorption is desired, the term "substantially no absorption" may allow for nominal amounts of absorption of one or more components of the bodily fluids into the wicking material 104 *(e.g*., absorbency), such as less than about 10 wt% of the dry weight of the wicking material 104, less than about 7 wt%, less than about 5 wt%, less than about 3 wt%, less than about 2 wt%, less than about 1 wt%, or less than about 0.5 wt% of the dry weight of the wicking material 104.

Additional examples of fluid collection assemblies are disclosed in U.S. Patent No. 10,226,376 filed on June 1, 2017.

As will be discussed in more detail with regards to **FIG. 5****,** the fluid collection assembly 100 may be in fluid communication with a fluid storage container and a vacuum source. For example, the port outlet 118 may be coupled to at least one second tube 158 and the second tube 158 may fluidly couple the fluid collection assembly 100 to the fluid storage container and the vacuum source. It is noted that the second tube 158 may be omitted, for example, when the sample port 110 is directly connected to a fluid storage container (*e.g.,* fluid storage container 566 of **FIG. 5**).

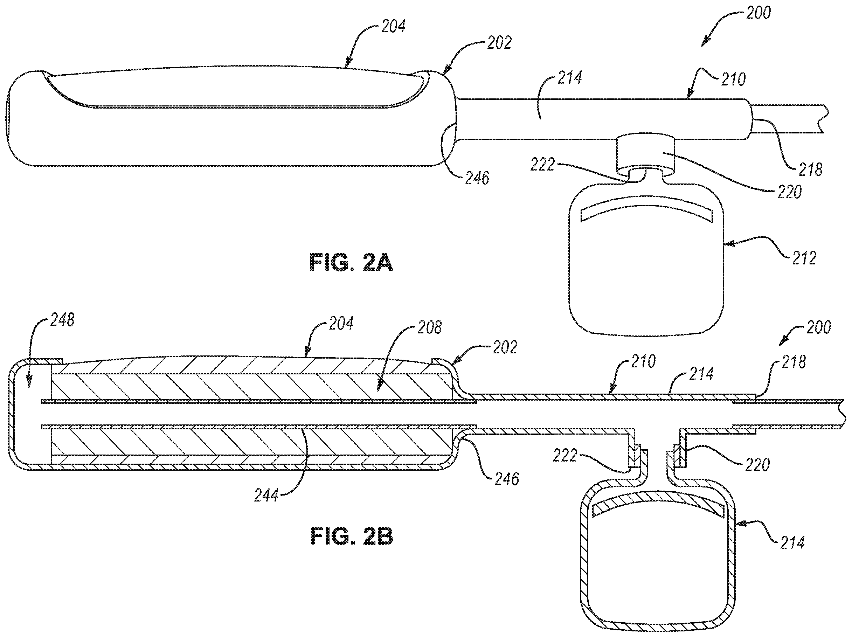

Although the sample port 110 is illustrated as being distinct from the fluid impermeable barrier 102, in some embodiments, the sample port 110 may be integrally formed with the fluid impermeable barrier 102. For example, **FIG. 2A** is a schematic isometric view of a fluid collection assembly 200 that includes a sample port 210 integrally formed with a fluid impermeable barrier 202, according to an embodiment. **FIG. 2B** is a schematic cross-sectional view of the fluid collection assembly 200, according to an embodiment. Except as otherwise disclosed herein, the fluid collection assembly 200 is the same as or substantially similar to any of the fluid collection assemblies disclosed herein. For example, the fluid collection assembly 200 includes the fluid impermeable barrier 202 defining a chamber 208, at least one wicking material 204 disposed in the chamber 208, the sample port 210, and a sample container 212.

As previously discussed, the fluid impermeable barrier 202 and the sample port 210 are integrally formed together. For example, the fluid impermeable barrier 202 defines an barrier outlet 246 that allows any bodily fluids present in the chamber 208 to leave the chamber 208. The sample port 210 includes at least one first wall 214 extending from the barrier outlet 246. The first wall 214 is integrally formed with the portion of the fluid impermeable barrier 202 that defines the barrier outlet 246. The first wall 214 defines a port outlet 218 and a first channel 226 extending between the barrier outlet 246 (*i*.*e*., the port inlet) and the port outlet 218. The sample port 210 may also include at least one second wall 220 extending from the first wall 214 to an attachment port 222 that is configured to be coupled to the sample container 212.

In an embodiment, referring to **FIG. 2B****,** the fluid collection assembly 200 may include at least one tube 244 extending from the barrier outlet 246 to a location at or near a fluid reservoir 248. The tube 244 facilitate removal of any bodily fluids in the chamber 208 and/or the fluid reservoir 248. In an embodiment, as illustrated, the tube 244 is distinct from the fluid impermeable barrier 202 and the sample port 210. In an embodiment, the tube 244 may be integrally formed with the fluid impermeable barrier 202 and the sample port 210.

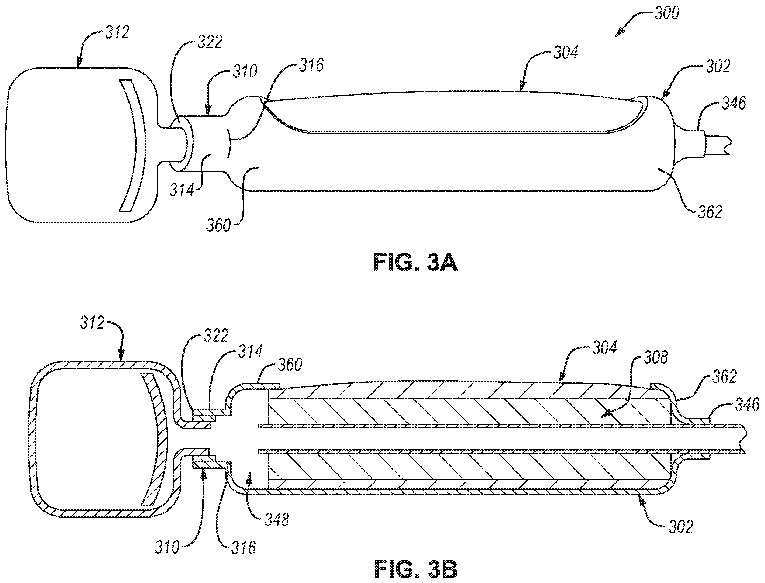

The sample ports disclosed herein may be integrally formed with other portions of the fluid impermeable barrier. For example, **FIG. 3A** is a schematic isometric view of a fluid collection assembly 300 that includes a sample port 310 integrally formed with a fluid impermeable barrier 302, according to an embodiment. **FIG. 3B** is a schematic cross-sectional view of the fluid collection assembly 300, according to an embodiment. Except as otherwise disclosed herein, the fluid collection assembly 300 is the same as or substantially similar to any of the fluid collection assemblies disclosed herein. For example, the fluid collection assembly 300 includes the fluid impermeable barrier 302 defining a chamber 308, at least one wicking material 304 disposed in the chamber 308, the sample port 310, and a sample container 312.

The fluid impermeable barrier 302 includes a first tip 360 and a second tip 362 opposite the first tip 360. The first tip 360 may define the fluid reservoir 348 and the second tip 362 may define the barrier outlet 346. The sample port 310 may be integrally formed with a portion of the fluid impermeable barrier 302 at or near the first tip 360. As such, the sample port 310 may be receive bodily fluids that are moving towards and/or are stored in the fluid reservoir 348.

The sample port 310 defines a port inlet 316 formed in a portion of the fluid impermeable barrier 302 at or near the first tip 360. The sample port 310 includes at least one wall 314 defining an attachment port 322. The wall 314 extends between the port inlet 316 and the attachment port 322. The attachment port 322 is couplable to the sample container 312. In the illustrated embodiment, the sample port 310 does not define a port outlet. However, it is noted that the sample port 310 may define a port outlet.

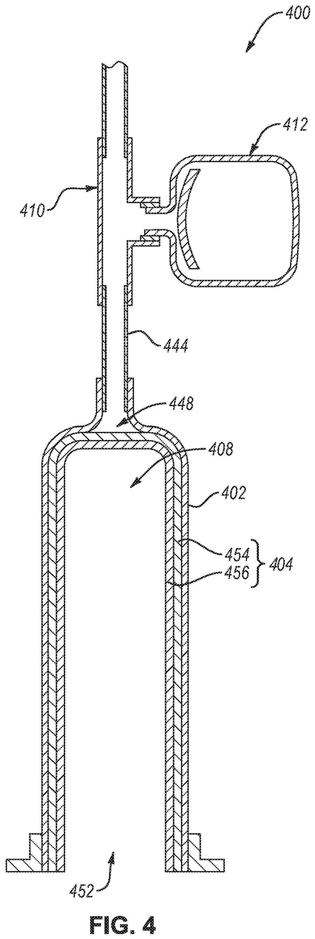

The fluid collection assemblies illustrated in **FIGS. 1A-3B** are configured to have the openings thereof positioned adjacent to a urethra (*e.g.,* a female urethra or a buried penis). However, the principles and concepts disclosed herein may be used with fluid collection assemblies that are configured to have the openings thereof receive a urethra (*e.g.,* male urethra) such that the urethra is disposed in the chamber. For example, **FIG. 4** is a schematic cross-sectional view of a fluid collection assembly 400 defining an opening 452 that is configured to receive a urethra, according to an embodiment. Except as otherwise disclosed herein, the fluid collection assembly 400 may be the same or substantially similar to any of the fluid collection assemblies disclosed herein. For example, the fluid collection assembly 400 may include a fluid impermeable barrier 402 defining a chamber 408, an barrier outlet 446, a fluid reservoir 448, and the opening 452. The fluid collection assembly 400 may also include at least one wicking material 404 (*e.g.,* a fluid permeable support 454 and a fluid permeable membrane 456) disposed in the chamber 408. The fluid collection assembly 400 may also include a sample port 410 and a sample container 412.

As previously discussed, the opening 452 is configured to receive the urethra. As such, the wicking material 404 does not extend across the opening 452. Instead, the wicking material 404 may exhibit a shape that corresponds to the shape of the fluid impermeable barrier 402 such that a portion of the chamber 408 is substantially unoccupied and the substantially unoccupied portion of the chamber 408 may receive the urethra.

In an embodiment, as illustrated, the sample port 410 is distinct from and, optionally, may be spaced from fluid impermeable barrier 402. As such, the sample port 410 may be the same or substantially similar to the sample port 110 illustrated in **FIGS. 1A** and **IB.** Further, in such an embodiment, the fluid collection assembly 400 may include at least one tube 444 extending between the sample port 410 and the barrier outlet 446 of the fluid impermeable barrier 402 such that the sample port 410 is in fluid communication with the chamber 408. In an embodiment, the sample port 410 may be integrally formed with the fluid impermeable barrier 402. In an example, the sample port 410 may be integrally formed with the portion of the fluid impermeable barrier 402 that defines the barrier outlet 446 and, thus, may be the same or substantially similar to the sample port 210 illustrated in **FIGS. 2A** and **2B****.** In an example, the sample port 410 may be integrally formed with a portion of the fluid impermeable barrier 402 that is spaced from the barrier outlet 446 and, thus, may be the same or substantially similar to the sample port 310 illustrated in **FIGS. 3A** and **3B****.**

Additional examples of fluid collection assemblies that include an opening that is configured to receive a urethra that may include the features and principles disclosed herein are disclosed in U.S. Patent Application No. 16/433,773 filed on June 6, 2019.

**FIG. 5** is a schematic illustration of fluid collection system 564 that includes a fluid collection assembly 500, according to an embodiment. The fluid collection assembly 500 may include any of the fluid collection assemblies disclosed herein. The fluid collection assembly 500 may be in fluid communication with a fluid storage container 566 via, for example, at least one first tube 558 *(e.g.,* second tube 158 of **FIGS. 1A** and **IB**). However, it is noted that the first tube 558 may be omitted from the system 564, for example, when the sample port of the fluid collection device 500 is directly connected to an inlet of the fluid storage container 566. The fluid storage container 566 may include an enclosed container for storing fluids, such as urine. For example, the fluid storage container 566 may include a pouch, a jar, a canister, a basin, or the like. The fluid storage container 566 may be constructed of one or more of glass, polymer(s) *(e.g*., polyethylene, polystyrene, polypropylene, polycarbonate, etc.), silicone, or any other fluid impermeable material. For example, the fluid storage container 566 may include a polymer bag or polymer canister.

The fluid storage container 566 is positioned downstream from the fluid collection assembly 500. The fluid storage container 566 may be in fluid communication with a vacuum source 568 via at least one second tube 570. The vacuum source 568 may include a vacuum pump, a hand operated suction device (*e.g.,* suction bulb), a vacuum line (e.g., wall mounted vacuum line available in hospital rooms), or the like. The vacuum source 568 may be selectively operable, such as having an on/off switch for controlled use. The vacuum source 568 is positioned downstream from the fluid storage container 566. During operation, the vacuum source 568 provides a suction force to the fluid collection assembly 500. The suction force draws fluid into the chamber and towards the first tube 558. The fluid that enters the first tube 558 is pulled by the suction force towards the fluid storage container 566 such that the fluid storage container 566 receives the fluid. The fluid storage container 566 may be configured to inhibit the fluid from flowing from the fluid storage container 566 to the vacuum source 568.

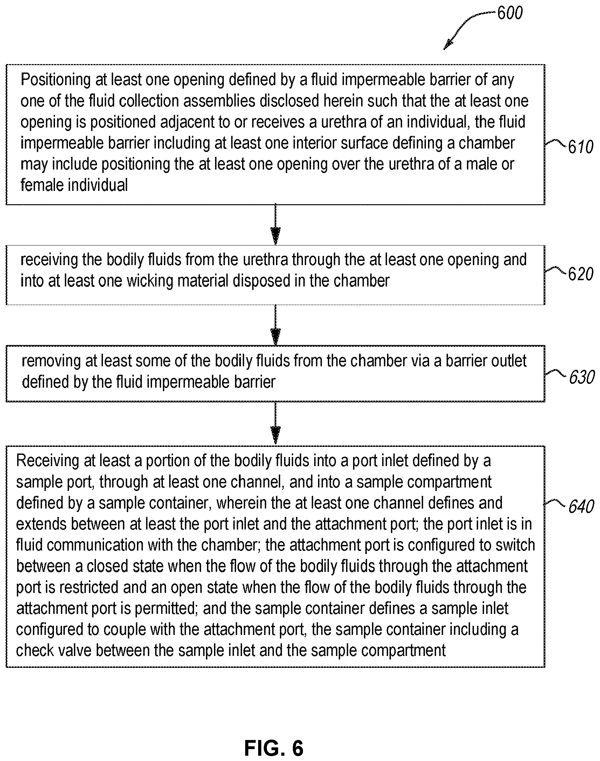

**FIG. 6** is a flow chart for a method 600 for collecting a sample of bodily fluids, according to at least some embodiments. The method 600 includes block 610 of positioning at least one opening defined by a fluid impermeable barrier of any one of the fluid collection assemblies disclosed herein such that the at least one opening is positioned adjacent to or receives a urethra of an individual, the fluid impermeable barrier including at least one interior surface defining a chamber. The method 600 includes block 620 of receiving the bodily fluids from the urethra through the at least one opening and into at least one wicking material disposed in the chamber. The method 600 includes block 630 of removing at least some of the bodily fluids from the chamber via a barrier outlet defined by the fluid impermeable barrier. The method 600 includes block 640 of receiving at least a portion of the bodily fluids into a port inlet defined by a sample port, through at least one channel, and into a sample compartment defined by a sample container, wherein the at least one channel defines and extends between at least the port inlet and the attachment port; the port inlet is in fluid communication with the chamber; the attachment port is configured to switch between a closed state when the flow of the bodily fluids through the attachment port is restricted and an open state when the flow of the bodily fluids through the attachment port is permitted; and the sample container defines a sample inlet configured to couple with the attachment port, the sample container including a check valve between the sample inlet and the sample compartment.

Block 610 of positioning at least one opening defined by a fluid impermeable barrier of any one of the fluid collection assemblies disclosed herein such that the at least one opening is positioned adjacent to or receives a urethra of an individual, the fluid impermeable barrier including at least one interior surface defining a chamber may include positioning the at least one opening over the urethra of a male or female individual. For example, positioning the at least one opening over the urethra of a male may include inserting the penis of a male in the chamber of the fluid collection assembly. Positioning the at least one opening over the urethra of a female may include positioning the opening on the labia of the female individual.

Block 620 of receiving the bodily fluids from the urethra through the at least one opening and into at least one wicking material disposed in the chamber may include wicking the urine from the individual into the chamber of the fluid collection.

Block 630 of removing at least some of the bodily fluids from the chamber via a barrier outlet defined by the fluid impermeable barrier includes utilizing one of gravity, a pump, or a vacuum source to remove the bodily fluids. Removing at least some of the bodily fluids from the chamber via a barrier outlet defined by the fluid impermeable barrier may include one or more of opening a valve, connecting the fluid collection assembly to the vacuum source or pump, turning on the vacuum source, or operating the pump.

Block 640 of receiving at least a portion of the bodily fluids into a port inlet defined by a sample port, through at least one channel, and into a sample compartment defined by a sample container may include utilizing any of the sample ports and sample containers disclosed herein. For example, the at least one channel may define and extend between at least the port inlet and the attachment port, the port inlet may be in fluid communication with the chamber, the attachment port may switch between a closed state when the flow of the bodily fluids through the attachment port is restricted and an open state when the flow of the bodily fluids through the attachment port is permitted, and the sample container may defines a sample inlet sized and shaped to couple with the attachment port, the sample container may include a check valve between the sample inlet and the sample compartment.

The method 600 may include coupling the sample inlet to the attachment port, such as before receiving at least a portion of the bodily fluids into the port inlet. Coupling the sample inlet to the attachment port may switch the attachment port from the closed state to the open state thereof. The method 600 may include decoupling the sample inlet from the attachment port, such as after receiving at least a portion of the bodily fluids into a port inlet. Decoupling the sample inlet from the attachment port may switch the attachment port from the open state to the closed state thereof.

While various aspects and embodiments have been disclosed herein, other aspects and embodiments are contemplated. The various aspects and embodiments disclosed herein are for purposes of illustration and are not intended to be limiting.

Terms of degree (*e.g.,* "about," "substantially," "generally," etc.) indicate structurally or functionally insignificant variations. In an example, when the term of degree is included with a term indicating quantity, the term of degree is interpreted to mean ± 10%, ±5%, +2% or 0% of the term indicating quantity. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc.

## Claims

1. A fluid collection assembly (100), comprising:
a fluid impermeable barrier (102) including at least one interior surface defining a chamber (108), the fluid impermeable barrier defining at least one opening and a barrier outlet; and
at least one wicking material (104) disposed in the chamber;
**characterized by**:
a sample port (110) defining at least one channel (124) extending between at least a port inlet (116) and an attachment port (122) thereof, the attachment port being configured to switch between a closed state when a flow of bodily fluids through the attachment port is restricted and an open state when the flow of the bodily fluids through the attachment port is permitted; and
a sample container (112) defining a sample compartment and a sample inlet (113) configured to couple with the attachment port, the sample container including a check valve (134) between the sample inlet and the sample compartment.

2. The fluid collection assembly of claim 1, wherein the sample port is integrally formed with the fluid impermeable barrier.

3. The fluid collection assembly of any of claims 1 or 2, wherein:
the fluid impermeable barrier defines a fluid reservoir (148) at a tip of the fluid impermeable barrier; and
the port inlet formed in or near the tip of the fluid impermeable barrier.

4. The fluid collection assembly of any of claims 1-3, wherein the sample port extends from the barrier outlet.

5. The fluid collection assembly of any of claims 1, 3, or 4, wherein the sample port is distinct from the fluid impermeable barrier; and
further comprising at least one tube extending from the barrier outlet to the port inlet.

6. The fluid collection assembly of any of claims 1-5, wherein the at least one channel defines a port outlet positioned downstream from the port inlet, and wherein the at least one channel includes a first channel extending from the port inlet to the port outlet and a second channel extending from the first channel to the attachment port.

7. The fluid collection assembly of any one of claims 1-6, wherein the sample port includes a valve (128) that is configured to switch the attachment port between the closed state and the open state thereof.

8. The fluid collection assembly of claim 7, wherein the valve is configured to cause the attachment port to be in the closed state when the sample inlet is not coupled to the attachment port and in the open state when the sample inlet is coupled to the attachment port.

9. The fluid collection assembly of any one of claims 7 or 8, wherein the valve and the sample inlet form a luer lock or a luer slip connection therebetween.

10. The fluid collection assembly of any one of claims 1-9, wherein the sample port includes at least one seal across the attachment port and wherein coupling the sample inlet to the attachment port breaks or otherwise damages the at least one seal.

11. The fluid collection assembly of any one of claims 1-10, wherein the check valve includes a curved barrier extending substantially between a portion of at least one interior surface of the sample port to an opposing portion of the at least one interior surface.

12. A fluid collection system, comprising:
any of the fluid collection assemblies of claims 1-10;
a fluid storage container positioned downstream from the fluid collection assembly; and
a vacuum source positioned downstream from the fluid storage container.

13. The fluid collection system of claim 12, wherein the fluid storage container includes a pouch, ajar, or a canister.

14. The fluid collection system of any one of claims 12 or 13, wherein the fluid storage container includes one or more of a polymer or a glass.

15. The fluid collection system of any one of claims 12-14, wherein the vacuum source includes a vacuum pump, a hand operated suction device, or a vacuum line.

## Patentansprüche

1. Fluidsammelanordnung (100), umfassend:
eine fluidundurchlässige Barriere (102), die mindestens eine Innenfläche beinhaltet, die eine Kammer (108) definiert, wobei die fluidundurchlässige Barriere mindestens eine Öffnung und einen Barrierenauslass definiert; und
mindestens ein Saugmaterial (104), das in der Kammer angeordnet ist;
**gekennzeichnet durch**:
einen Probenport (110), der mindestens einen Kanal (124) definiert, der sich zwischen mindestens einem Porteinlass (116) und einem Befestigungsport (122) davon erstreckt, wobei der Befestigungsport ausgelegt ist, zwischen einem geschlossenen Zustand, in dem ein Strom von Körperfluida durch den Befestigungsport hindurch begrenzt ist, und einem offenen Zustand, in dem der Strom von Körperfluida durch den Befestigungsport hindurch erlaubt ist, umzuschalten; und
einen Probenbehälter (112), der ein Probenfach und einen Probeneinlass (113) definiert, der ausgestaltet ist, an den Befestigungsport gekoppelt zu werden, wobei der Probenbehälter ein Rückschlagventil (134) zwischen dem Probeneinlass und dem Probenfach beinhaltet.

2. Fluidsammelanordnung nach Anspruch 1, wobei der Probenport in einem Stück mit der fluidundurchlässigen Barriere gebildet ist.

3. Fluidsammelanordnung nach einem der Ansprüche 1 oder 2, wobei:
die fluidundurchlässige Barriere ein Fluidreservoir (148) an einer Spitze der fluidundurchlässigen Barriere definiert; und
der Porteinlass in oder nahe der Spitze der fluidundurchlässigen Barriere gebildet ist.

4. Fluidsammelanordnung nach einem der Ansprüche 1-3, wobei sich der Probenport von dem Barrierenauslass erstreckt.

5. Fluidsammelanordnung nach einem der Ansprüche 1, 3 oder 4, wobei der Probenport sich von der fluidundurchlässigen Barriere unterscheidet; und
weiter umfassend mindestens ein Rohr, das sich von dem Barrierenauslass zu dem Porteinlass erstreckt.

6. Fluidsammelanordnung nach einem der Ansprüche 1-5, wobei der mindestens eine Kanal einen Portauslass definiert, der stromabwärts von dem Porteinlass positioniert ist, und wobei der mindestens eine Kanal einen ersten Kanal, der sich von dem Porteinlass zu dem Portauslass erstreckt, und einen zweiten Kanal, der sich von dem ersten Kanal zu dem Befestigungsport erstreckt, beinhaltet.

7. Fluidsammelanordnung nach einem der Ansprüche 1-6, wobei der Probenport ein Ventil (128) beinhaltet, das ausgelegt ist, den Befestigungsport zwischen seinem geschlossenen Zustand und seinem offenen Zustand umzuschalten.

8. Fluidsammelanordnung nach Anspruch 7, wobei das Ventil ausgestaltet ist, den Befestigungsport zu veranlassen, im geschlossenen Zustand zu sein, wenn der Probeneinlass nicht an den Befestigungsport gekoppelt ist, und im offenen Zustand, wenn der Probeneinlass an den Befestigungsport gekoppelt ist.

9. Fluidsammelanordnung nach einem der Ansprüche 7 oder 8, wobei das Ventil und der Probeneinlass einen Luer-Verschluss oder eine Luer-Gleitverbindung dazwischen bilden.

10. Fluidsammelanordnung nach einem der Ansprüche 1-9, wobei der Probenport mindestens eine Dichtung über den Befestigungsport beinhaltet und wobei Koppeln des Probeneinlasses an den Befestigungsport die mindestens eine Dichtung bricht oder auf andere Weise beschädigt.

11. Fluidsammelanordnung nach einem der Ansprüche 1-10, wobei das Rückschlagventil eine gekrümmte Barriere beinhaltet, die sich im Wesentlichen zwischen einem Abschnitt mindestens einer Innenfläche des Probenports zu einem gegenüberliegenden Abschnitt der mindestens einen Innenfläche erstreckt.

12. Fluidsammelsystem, umfassend:
eine der Fluidsammelanordnungen nach Ansprüchen 1-10;
einen Fluidlagerbehälter, der stromabwärts von der Fluidsammelanordnung positioniert ist; und
eine Vakuumquelle, die stromabwärts von dem Fluidlagerbehälter positioniert ist.

13. Fluidsammelsystem nach Anspruch 12, wobei der Fluidlagerbehälter einen Beutel, einen Krug oder einen Kanister beinhaltet.

14. Fluidsammelsystem nach einem der Ansprüche 12 oder 13, wobei der Fluidlagerbehälter eines oder mehrere von einem Polymer oder einem Glas beinhaltet.

15. Fluidsammelsystem nach einem der Ansprüche 12-14, wobei die Vakuumquelle eine Vakuumpumpe, eine handbetriebene Saugvorrichtung oder eine Vakuumleitung beinhaltet.

## Revendications

1. Ensemble de collecte de fluide (100), comprenant :
une barrière imperméable aux fluides (102) incluant au moins une surface intérieure définissant une chambre (108), la barrière imperméable aux fluides définissant au moins une ouverture et une sortie de barrière ; et
au moins un matériau à effet de mèche (104) disposé dans la chambre ;
**caractérisé par** :
un orifice d'échantillon (110) définissant au moins un canal (124) s'étendant entre au moins une entrée d'orifice (116) et un orifice de fixation (122) de celui-ci, l'orifice de fixation étant configuré pour basculer entre un état fermé lorsqu'un écoulement de fluides corporels à travers l'orifice de fixation est restreint et un état ouvert lorsque l'écoulement des fluides corporels à travers l'orifice de fixation est autorisé ; et
un récipient d'échantillon (112) définissant un compartiment d'échantillon et une entrée d'échantillon (113) configurée pour se coupler avec l'orifice de fixation, le récipient d'échantillon incluant un clapet anti-retour (134) entre l'entrée d'échantillon et le compartiment d'échantillon.

2. Ensemble de collecte de fluide selon la revendication 1, dans lequel l'orifice d'échantillon est formé d'un seul tenant avec la barrière imperméable aux fluides.

3. Ensemble de collecte de fluide selon l'une quelconque des revendications 1 ou 2, dans lequel :
la barrière imperméable aux fluides définit un réservoir de fluide (148) au niveau d'une pointe de la barrière imperméable aux fluides ; et
l'orifice d'entrée est formé dans ou près de la pointe de la barrière imperméable aux fluides.

4. Ensemble de collecte de fluide selon l'une quelconque des revendications 1 à 3, dans lequel l'orifice d'échantillon s'étend depuis la sortie de barrière.

5. Ensemble de collecte de fluide selon l'une quelconque des revendications 1, 3 ou 4, dans lequel l'orifice d'échantillon est distinct de la barrière imperméable aux fluides ; et
comprenant en outre au moins un tube s'étendant de la sortie de barrière à l'entrée d'orifice.

6. Ensemble de collecte de fluide selon l'une quelconque des revendications 1 à 5, dans lequel le au moins un canal définit une sortie d'orifice positionnée en aval de l'entrée d'orifice, et dans lequel le au moins un canal inclut un premier canal s'étendant de l'entrée d'orifice à la sortie d'orifice et un second canal s'étendant du premier canal à l'orifice de fixation.

7. Ensemble de collecte de fluide selon l'une quelconque des revendications 1 à 6, dans lequel
l'orifice d'échantillon inclut un clapet (128) qui est configuré pour commuter l'orifice de fixation entre l'état fermé et l'état ouvert de celui-ci.

8. Ensemble de collecte de fluide selon la revendication 7, dans lequel le clapet est configuré pour amener l'orifice de fixation à être dans l'état fermé lorsque l'entrée d'échantillon n'est pas couplée à l'orifice de fixation et dans l'état ouvert lorsque l'entrée d'échantillon est couplée à l'orifice de fixation.

9. Ensemble de collecte de fluide selon l'une quelconque des revendications 7 ou 8, dans lequel le clapet et l'entrée d'échantillon forment un raccord de type luer ou une connexion à glissement de type luer entre ceux-ci.

10. Ensemble de collecte de fluide selon l'une quelconque des revendications 1 à 9, dans lequel l'orifice d'échantillon inclut au moins un joint d'étanchéité à travers l'orifice de fixation et dans lequel le couplage de l'entrée d'échantillon à l'orifice de fixation rompt ou détériore d'une autre manière le au moins un joint d'étanchéité.

11. Ensemble de collecte de fluide selon l'une quelconque des revendications 1 à 10, dans lequel le clapet anti-retour inclut une barrière incurvée s'étendant sensiblement entre une partie d'au moins une surface intérieure de l'orifice d'échantillon et une partie opposée de la au moins une surface intérieure.

12. Système de collecte de fluide, comprenant :
l'un quelconque des ensembles de collecte de fluide des revendications 1 à 10 ;
un récipient de stockage de fluide positionné en aval de l'ensemble de collecte de fluide ; et
une source de vide positionnée en aval du récipient de stockage de fluide.

13. Système de collecte de fluide selon la revendication 12, dans lequel le récipient de stockage de fluide inclut une poche, un bocal ou une cartouche.

14. Système de collecte de fluide selon l'une quelconque des revendications 12 ou 13, dans lequel le récipient de stockage de fluide inclut un ou plusieurs parmi un polymère ou un verre.

15. Système de collecte de fluide selon l'une quelconque des revendications 12 à 14, dans lequel la source de vide inclut une pompe à vide, un dispositif d'aspiration manuel, ou une conduite de vide.
